# EUROPEAN PATENT APPLICATION

(11) **EP 4 166 171 A1**
(43) Date of publication of application: **19.04.2023**
(21) Application number: 21202716.3
(22) Date of filing: 14.10.2021
(51) Int. Cl.: A61M 5/24, A61M 5/31

(54) **PRE-ASSEMBLED CARTRIDGE UNIT FOR INJECTION DEVICE**

(71) Applicant: Ypsomed AG, 3401 Burgdorf (CH)
(72) Inventor: BOSSHARD, Simon Martin, 3006 Bern (CH); SCHEURER, Simon, 3006 Bern (CH); SCHRUL, Christian, 3400 Burgdorf (CH); BRÜGGER, Martin, 3065 Bolligen (CH)
(74) Representative: Meier Obertüfer, Jürg

(57) **Abstract**

The present invention relates to a pre-assembled reservoir unit (1) for attachment to a drive unit (50, 60) of a drug delivery device (5, 6) for dispensing a drug from a reservoir (4). The reservoir unit (1) comprises the reservoir (4), a reservoir holder (3) and a mechanics holder (10) defining a longitudinal axis and connected to the reservoir holder (3), wherein the mechanics holder (10) comprises a housing connecting portion (14) for coaxially attaching the mechanics holder (10) to a housing (51, 62) of the drive unit along the longitudinal axis. The reservoir unit (1) further includes a plunger rod (30) that is in threaded or splined engagement with the mechanics holder (10).

## Description

### FIELD OF THE INVENTION

The present invention relates to injection devices or medicament delivery devices for injecting, delivering, administering, infusing or dispensing substances and/or liquids such as insulin, hormone preparations or vaccines. It departs from a drug delivery device comprising a drive unit with a manual or automatic drive including a movable drive. The delivery device further comprising a pre-assembled reservoir unit attachable to the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

There are several types of drug delivery devices known in the art. Disposable delivery devices are adapted to deliver medication from a container such as a pre-filled cartridge or syringe that is not intended to be replaced or refilled by the patient. Reusable, semi-disposable, or hybrid delivery devices have components that may be replaced by the patient, or a cartridge that may be refilled, while some components of the device may be reused with the replaced or refilled drug container. WO 2019/112886 A1 discloses a delivery device comprising a disposable cassette having a cartridge housing and a reusable module having a main housing. A drive ribbon is disposed within the cartridge housing and the cartridge holding a medication is mounted laterally on the cartridge housing of the disposable cassette. A drive member mechanism of the drive ribbon is disposed in the main housing of the reusable module.

WO 2019/158372 A1 discloses delivery device with a back-end reusable power pack unit and a front-end disposable drug cassette unit including a syringe or a cartridge. The drug cassette unit is axially inserted into the reusable unit and being fixed by snap-connection due to expanding of a flexible parts. A medicament container is spaced within a housing of the disposable unit. A drive unit provided with a plunger rod and a battery is mounted on a frame adjacent to a closed end of the reusable unit.

WO19122946 A1 discloses an automatic injector device that comprises a single-use disposable assembly and a reusable assembly. The disposable assembly comprises a housing and a syringe within the housing. The reusable assembly comprises a drive motor with a gear, a power supply, a programmable control system and a screw threaded piston rod. After an injection the disposable assembly can be detached from the reusable assembly and disposed of whilst the reusable assembly can be re-used after reconnecting with a new, unused, disposable assembly.

These prior art approaches suffer from the fact that the drive assembly has to be precisely adjusted or aligned to the attached reservoir assembly to enable a reliable dose dispensing and to reduce the risk of an inaccuracies. Often a cumbersome priming or preparing operation has to be carried out by the user after attaching a new reservoir unit to a drive unit to bring a distal tip or flange of the plunger rod into contact with a plunger inside a reservoir. Some reservoir assemblies and drive assemblies are bulky (parallel attachment as shown in WO 2019/112886) and require a complex assembly and/or operation.

### DESCRIPTION OF THE INVENTION

It is an objective of the invention to allow an accurate dose dispensing with a drug delivery device wherein a cost-effective manufacturing, storing and final assembly of a drug delivery device is provided.

This objective is achieved by a drug delivery device and a reservoir unit according to the independent claims. Preferred embodiments are evident from the dependent claims.

The invention relates to a drug delivery device for dispensing a liquid drug from a reservoir. The drug delivery device comprises a drive unit (preferably a reusable unit) that includes a housing defining a longitudinal axis and a manual or automatic drive arranged at least partially inside the housing. The drive unit includes further a drive member movable relative to the housing for driving a plunger rod of a reservoir unit for dispensing the drug.

The drug delivery device further comprises a pre-assembled reservoir unit (preferably a disposable unit) releasably attachable or non-releasably attached to the drive unit along the longitudinal axis. The attachement along the longitudinal axis does not exclude an additional screw or bayonet movement of the reservoir unit relative to the drive unit. The reservoir unit includes a reservoir, a reservoir holder and a mechanics holder connected to the reservoir holder. The mechanics holder comprises a housing connecting portion for coaxially connecting or attaching the mechanics holder to the housing of the drive unit. The reservoir unit includes further the plunger rod that is in threaded or splined engagement with the mechanics holder preferably guided in the longitudinal direction in a thread or with a spline connection in the mechanics holder.

The movable drive member of the drive unit includes a coupling structure or a coupling element for coupling the drive member to the plunger rod such that a movement of the drive member is transferred to the plunger rod once the reservoir unit is attached to the drive unit.

The reservoir unit is pre-assembled. That means the mechanics holder, the reservoir holder with the reservoir and the plunger rod are previously assembled and can be handled and stored together as one assembled unit. That means the reservoir unit has no specific reservoir unit housing that remains visible once the reservoir unit is attached to the drive unit.

As the reservoir unit is assembled with the plunger rod the latter can be positioned relative to the reservoir and in particular relative to a movable plunger inside the reservoir at the manufacturer's site. The user does not have to align or otherwise position the plunger rod relative to the reservoir. Consequently, a priming operation, or any other an assembly or preparing step carried out by the user prior to dispensing the drug and potentially leading to inaccuracies can be avoided. Due to the high accurancy the reservoir unit according to the invention is thus in particular suitable for a 300 unit/mL cartridge (U300).

Besides that, a flange or a distal portion of the plunger rod having contact with the reservoir does not have to be cleaned or otherwise prepared for a dispensing after replacement of a reservoir unit as the plunger rod is discarded together with the reservoir and each reservoir unit has its own new plunger rod. The user does not have to reset or otherwise move the plunger rod when attaching a new reservoir unit to the drive unit.

The plunger rod is thus only used for the dispensing with one reservoir. Hence, the lifetime of the plunger rod is short compared to prior art mechanisms with a plunger rod used for a plurality of reservoirs. That allows for a cost efficient design and manufacturing in particular regarding a thread or connecting elements of the plunger rod.

Furthermore, the dosing accuracy with the mechanics holder according to the invention is improved as the mechanics holder couples or holds the reservoir holder with the reservoir, the plunger rod and the housing of the drive unit in place and hence acts as a one piece interface member reliably holding these parts in a defined position relative to each other.

What is more, the mechanics holder is separate from a housing or an outer shell of the drug delivery device. This is in contrast to prior art solutions and allows to decouple the mechanics holder and the plunger rod from the manual or automatic drive and thus from forces introduced during dose setting or dose dispensing and acting on the drive unit housing or the drive member other than the dispensing torque. Thus, impacts from the drive unit to the reservoir unit (in particular to the plunger rod and reservoir) and potentially increasing the risk of dose inaccuracies can be significantly reduced.

In other words, the transmission of user exerted forces from the drive unit to the plunger rod can be almost completely avoided as the plunger rod is not guided in or engaged to the housing of drive unit. According to the invention the plunger rod is in splined or threaded engagement exclusively with the mechanics holder of the attached reservoir unit. While a driving movement of a drive member of the drive unit has to be transmitted to the plunger rod such a movement may be clearly restricted, for example, to an exclusive rotational movement (no shifting) or an exclusive shifting movement. This contributes to reducing the impact of driving forces.

Besides that, the mechanics holder and the housing both can be individually designed with specific properties like material (e.g. stiffness), dimensions and/or design and appearance. By way of example, the housing may be made of a biodegradable plastics as the housing usually constitutes the largest portion of the device. In contrast, the mechanics holder may be made of a plastic having a high rigidity or a minimum elasticity.

The drug delivery device may be a reusable, a disposable, a semi-disposable (also named as semi-reusable) delivery device. A semi-disposable delivery device comprises units, components or sub-assemblies that are discarded (disposable part) after a dose dispensing or if the reservoir is empty. Additionally, the semi-disposable device includes units, components or sub-assemblies that can be reused (reusable part) for a plurality of dose dispensing operations.

The reservoir unit is preferably replaced after one or more dose dispensing events or after the drug of the reservoir is completely dispensed. The reservoir unit is thus preferably a disposable unit. In contrast, the drive unit is preferably used for several dose dispensing operations and for different reservoir units and hence forms or is part of a reusable unit.

The drug delivery device is preferably an injection device, in particular a pen-shaped injection device. Alternatively, the drug delivery device may be an infusion device.

The term "injection device" or "injector" refers to a device that is removed from the injection site after each medication event or drug delivery process. By contrast, an "infusion device" refers to a device with a cannula or needle that remains in the skin of the patient for a prolonged period of time, for example, several hours.

The mechanics holder couples or holds the reservoir holder, the reservoir, the plunger rod and the housing of the drive unit in place. The mechanics holder includes a housing coupling portion with one or more coupling elements (e. g. a snapper, latch, protrusion, tap, flap, nut, arm, bayonet, screw) to attach, connect or couple the mechanics holder coaxially to the housing of the drive unit along the longitudinal axis. That means the mechanics holder and the housing have the same longitudinal axis. This allows a space saving design with respect to the radial dimensions. That is easy to handle and is in particular advantageous as the user usually prefers a thin or elongated shape of the drug delivery device.

The mechanics holder is thus separated from the drive mechanism and does not support, hold or engage driving and/or dosing elements of the drive mechanism. These members are preferably located inside the housing of the drive unit.

The housing coupling portion is preferable arranged at a proximal portion or side of the mechanics holder. Additionally, the mechanics holder preferably includes a holder coupling portion arranged at a distal portion or at a distal end of the mechanics holder to attach the reservoir holder.

The mechanics holder preferably includes a guiding structure for the plunger rod. Non-limiting examples of such structure are a thread or one or more protrusion, nut or spline for linear or rotational guidance of the plunger rod.

According to the invention the reservoir unit is assembled with the plunger rod. The plunger rod is in threaded or splined engagement with the mechanics holder. Thus, the plunger rod includes a thread or a spline element. In case the plunger rod is splined to the mechanics holder the plunger rod may be shifted in a dispensing direction during dose dispensing.

The reservoir may be a container, cartridge (one or two chamber), vial or ampule adapted to contain the drug which can be dispensed out of the reservoir by the plunger rod. The reservoir is preferably a cartridge, in particular a non-refillable cartridge for single use. A cartridge does not integrally comprise any plunger rod or piston rod.

The reservoir unit may be releasably or non-releasably attached to the drive unit. In the latter case the pre-assembled reservoir unit may be assembled in a final step with drive unit to form a disposable device. That means both the reservoir unit and the drive unit are discarded after use or if the reservoir is empty.

In case the reservoir unit is releasably or non-permanently attachable to the drive unit it is non-destructibly detachable from the reservoir unit. In this case the user may replace the reservoir unit including the reservoir when the reservoir is empty or has to be replaced or if the user intends to change the drug.

The drive unit comprises an automatic drive or a manual drive requiring a user force to move the movable drive member for dispensing a dose.

If the drive unit is an automatic drive it may include for example an electric motor with a motor shaft or a biased actuation member such as a spring or a biased elastomer element. Furthermore, the automatic drive may include a power source such as, for example, a mechanical spring, a single use or rechargeable electric battery, a hydraulic drive or a gas carpule.

If the drive unit is a manual drive unit it is manually driven. The manual drive unit is preferably devoid of any automatic drive means such as a spring, a biased elastic element or any kind of motor. Preferably, the manual drive unit is exclusively hand-driven. That means a user has to push, shift, rotate or screw to move the drive member to dispense a dose.

The manual or automatic drive unit includes a drive member that can be operatively coupled or is connectable to the plunger rod when the drive unit is attached to the reservoir unit. The term "couple" or "connectable" includes embodiments with zero, one or more intermediate members between the drive member and the plunger rod. When the drive unit is attached to the reservoir unit the drive member is directly or indirectly, via one or more intermediate member coupled to the plunger rod. That means an advancing movement of the drive member is converted into an advancing movement of the plunger rod. The advancing movement of the plunger rod can be an axial shifting, a helical or a screw movement.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The mechanics holder is preferably made in one piece. That means the mechanics holder is monolithic. That allows a cost-efficient manufacturing and ensures that the plunger rod is precisely positioned relative to the reservoir holder (and thus to the reservoir) as interfaces between single parts and a tolerance chain can be avoided.

The mechanics holder is preferably injection molded and primarily made of plastics.

In a preferred embodiment the coupling structure of the drive member is a spline element for spline engagement and rotational coupling of the drive member with the plunger rod such that exclusively a rotational movement of the drive member is transferred to the plunger rod, when the drive unit is attached to the reservoir unit.

That ensures that exclusively rotational forces and movements of the drive member are transferred to the plunger rod and axial forces or axial movements of the drive member are not transferred to the plunger rod. Thus, the plunger rod is axially decoupled. Axial loads of the drive mechanism unintentionally affecting the movement of the plunger rod and potentially leading to dose dispensing inaccuracies can be avoided.

During attachement of the reservoir unit to the drive unit the plunger rod may be rotated to align the spline element and to allow a complete insertion of the plunger rod into the drive unit.

In an alternative embodiment, the drive member includes a thread or an axial coupling structure. In the latter case the plunger rod can be rotationally decoupled from the drive member and exclusively axial drive forces can be transferred to the plunger rod.

The mechanics holder is preferably non-releasably connectable to the reservoir holder at the manufacturer site during assembly of the drug delivery device. That means the connection cannot be released without destructing the parts. The mechanics holder may include a snap-fit element such that a holder connecting portion of the mechanics holder is connectable to the reservoir holder by a non-releasably snap-fit connection. The snap fit element may be, for example, a protrusion, arm, catch, snapper or a latching element. The reservoir holder may include a corresponding counter element (e.g. protrusion, arm, catch, snapper or the like) to enable the snap-fit connection.

Alternatively, the mechanics holder may be connected to the reservoir holder by a releasably connection such as a thread or a bayonet connection.

In a preferred embodiment the housing connecting portion includes a first connecting element on an outer surface of the mechanics holder and the housing includes a second connecting element arranged on an inside of an opening of the housing. The housing may be adapted to enclose at least a part of the mechanics holder, when the drive unit is attached to the reservoir unit. As the mechanics holder is partially enclosed by the housing a space saving arrangement is provided. Preferably at least the half of the length in the longitudinal direction of the mechanics holder is enclosed by the housing, more preferably at least 70% of the length in the longitudinal direction of the mechanics holder is enclosed by the housing.

The connecting element is for example a thread, a snapper, a protrusion, a nut or a catch. The connection may be a releasable connection or a non-releasable connection. If the device is a reusable or semi-disposable device the connection between the connecting element of the mechanics holder and the counter element of the housing of the drive unit is preferably a releasably connection (for example a thread, a bayonet or a releasable snap-fit connection).

Alternatively, the connecting elements may be arranged on another portion of the mechanics holder, for example in a middle of the longitudinal axis or at a proximal end of the mechanics holder.

Advantageously, the plunger rod is in threaded connection with the mechanics holder. That is, the mechanics holder includes an inner thread for the plunger rod and the plunger rod includes an outer thread. That means the plunger rod carries out a screw movement in a dispensing direction when the plunger rod is driven by the drive member. A dose can be thus reliably dispensed with a high accuracy.

Alternatively, the plunger rod may be guided in the mechanics holder by means of a splined engagement. In this case the plunger rod is exclusively advanced in the dispensing direction by a translation movement without rotation.

In a preferred embodiment the mechanics holder has a sleeve-shaped body including a distal end portion for attachment of the reservoir holder and a proximal portion for insertion into the housing of the drive unit.

The mechanics holder includes preferably a proximally arranged dose thread for a dose member, in particular a dose sleeve of the drive unit. This arrangement provides a compact design and as the dose sleeve is not engaged with the housing the latter can be design independently. Besides that, a further interface or separate part can be avoided by included thread. The thread in the mechanics holder is preferably an inner thread and the dose member comprises a corresponding outer thread.

Furthermore, the mechanics holder comprises preferably an integrally formed stop element to limit the movement of the dose member, in particular a dispensing stop to limit a maximum dispensing movement of the dose member in a dispensing direction. As the stop element is formed by the mechanics holder a further interface can be avoided.

In a preferred embodiment the mechanics holder includes a biasing element for biasing the reservoir in a dispensing direction against the reservoir holder in order to avoid a mechanical play between the reservoir, reservoir holder and mechanics holder. That means the reservoir is reliably and non-movably hold inside the reservoir holder and relative thereto. Hence, a high dose accuracy can be provided. The biasing element may further compensate manufacturer tolerances, a stretch or fatigue of material of the reservoir holder and/or the mechanics holder or a deformation during assembly of a needle on the reservoir holder.

Alternatively, the reservoir is hold inside the reservoir holder without a biasing element. In this case the reservoir holder and the mechanics holder have to be manufactured accurately to be able to tightly hold the reservoir in place.

Preferably, the biasing element is an integral part of the mechanics holder. The biasing element is thus monolithic integrated in a body of the mechanics holder. This is in particular advantageous if the mechanics holder is made of plastic and manufactured by injection molding as such an integral biasing element reduces the number of individual parts and contributes to an efficient manufacturing.

In a preferred embodiment the biasing element includes a compressible hollow chamber, which allows a compact design. A distal portion of the biasing member including the chamber may be connected to a body of the mechanics holder in case the biasing element is not integrally formed in the mechanics holder. The hollow chamber can be compressed by the reservoir. Due to the deformation of biasing member and elastic properties of the material a pre-tensioning force (biasing force) acts on the reservoir and thus biases the reservoir distally in dispensing direction against the reservoir holder.

Advantageously, the mechanics holder is primarily made of a first plastic material and the housing is primarily made of a second plastic material different than the first plastic material. Hence, a plastic with properties (stiffness, formability, durability, appearance) optimal for the mechanics holder and another plastic with other properties optimal for the housing can be selectively chosen. That allows to optimize these parts in view of the material. For example, the housing may be made of a plastic that allows to be printed and the mechanics holder may be a plastic that has suitable coefficient of friction (for example polyoxymethylene). As a further example, the housing may be made of biodegradable plastics. This is advantageous as the housing usually constitutes the major part of the device.

The term "primarily" means the holder and the housing are made for the most part made of the first or second plastic material. Preferably, the mechanics holder is of at least 80% made of the first plastic material and the housing is of at least 80% made of the second plastic material.

In case the drive unit is an automatic drive unit it includes preferably an electric motor (in particular for a reusable drive unit) or a pre-tensioned, re-tensionable or biased member (in particular for a disposable drive unit) for automatically moving the drive member. The re-tensionable member may be a spring or elastomer element that can be biased (charged) each time during dose dialing by the user. In this case the term "automatic" refers to the dispensing movement which is initiated by the biased spring or elastomer element. If the drive unit includes a motor it may be supplied with power from a power source in the drive unit such as, for example, a battery or a biased mechanical spring.

The drive member in turn drives the plunger rod in a dispensing direction to dispense the drug from the reservoir.

In an alternative embodiment the drive unit may be a manually operated drive unit. The manual drive may comprise a dose member for manually setting and correcting a dose and for manually rotating to dispense the dose. The dose member may be selectively and rotationally connectable to drive member during dose dispensing.

The manual drive unit is preferably devoid of any automatic drive means such as a spring, motor or biasing member. The manual drive can thus be manufactured at low cost.

The manually operated drive unit can be used to build either a disposable drive unit or a reusable drive unit. In contrast to devices known in the art the plunger rod does not have to be reset manually by the user as the plunger rod is assembled inside the reservoir unit and is discarded with the reservoir unit when the latter is replaced.

The invention further refers to the pre-assembled reservoir unit. The reservoir unit comprises the reservoir, a reservoir holder and the mechanics holder defining a longitudinal axis and connected to the reservoir holder, wherein the mechanics holder comprises a housing connecting portion for coaxially connecting the mechanics holder to a housing of the drive unit along the longitudinal axis. The reservoir unit further includes a plunger rod that is in threaded or splined engagement with the mechanics holder.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of a reservoir unit according to the invention;
- Fig.2: depicts a perspective view of a manual injection pen with the reservoir unit;
- Fig.3: depicts a perspective view of an automatic injection pen with the reservoir unit;
- Fig. 4: depicts a sectional view of the mechanics holder of the reservoir unit and
- Fig. 5: depicts a sectional view of the manual injection pen of figure 2.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

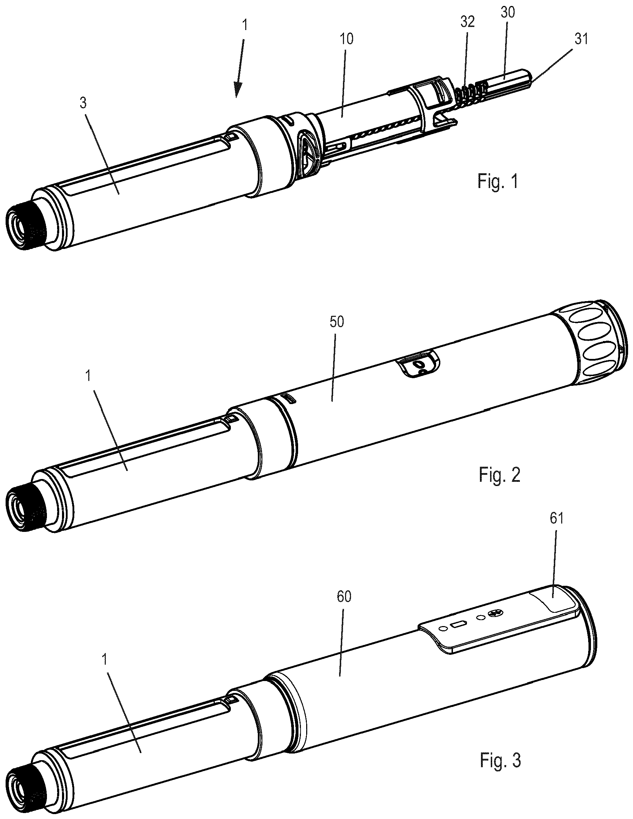

In the present description the term "distal" refers to the side where the needle is attached. This is on the left hand side in the figures 1 to 5. The term "proximal" refers to the opposite side and is on the right hand side in figures 1 to 5.

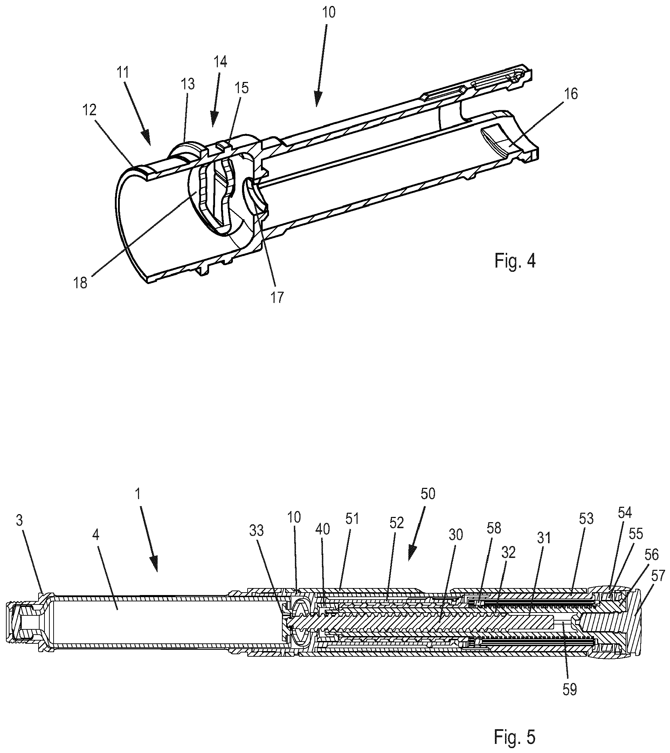

Figure 1 depicts a pre-assembled reservoir unit 1 according to the invention in a perspective view. The reservoir unit 1 is disposable and can be non-releasably attached to a disposable manual drive unit 50 as shown in the embodiment in figures or it can be releasably attached to a reusable automatic drive unit 60 as shown in the embodiment in figure 4.

The reservoir unit 1 can be produced, pre-assembled and stored separately from the drive units 50, 60. That is, single parts of the reservoir unit 1 may be produced by a device manufacturer and delivered to the drug or medication manufacturer. The latter inserts a filled cartridge in a reservoir holder and pre-assembles the single components to the pre-assembled reservoir unit 1.

Figure 2 and 3 show two different embodiments of a drug delivery device according to the invention. In the embodiment shown in figure 2 the reservoir unit 1 is attached to a manual drive unit 50 whereas an identical type of reservoir unit 1 is attached to a reusable automatic drive unit 60 that includes an electric motor for automatically dispensing a dose. Due to the modularity approach the same type of reservoir unit 1 can be used for different types of drive units to build different types of injection devices (a disposable manual injection device 5 and a semi-disposable automatic injection device 6).

The reservoir unit 1 and the manual drive unit 50 shown in figure 2 form a disposable and manually operated injection pen 5. The connection between the reservoir unit 1 and the manual drive unit 50 is non-releasable meaning that the reservoir unit 1 cannot be detached from the manual drive unit 50. Hence, the attachment of the reservoir unit 1 to the manual drive unit 50 is part of a final assembly. After use or when the reservoir is empty the manual injection pen 5 is discarded.

In an alternative embodiment the drive unit can be implemented as a reusable drive unit. Correspondingly, the connection between the reservoir unit 1 and the manually operated reusable drive unit is a releasable connection, for example a bayonet connection as described with respect to the following embodiment.

In contrast, in the embodiment shown in figure 3 the same type of reservoir unit 1 is releasably attached to a reusable automatic drive unit 60 constituting an automatic injection pen 6. In this embodiment only the reservoir unit 1 is detached and discarded after use or when reservoir is empty. The automatic drive unit 60 can be reused.

In the following the reservoir unit 1 as shown in detail in figure 1 is described in detail. The reservoir unit 1 includes a mechanics holder 10, a reservoir holder 3 connected to the mechanics holder 10, a reservoir (best shown in figure 5) in form of a cartridge 4 inside the reservoir holder 3 and a plunger rod 30 with a flange 33 pivotally mounted on a distal end of the plunger rod 30. A pen cap (not shown) can be mounted on a needle assembly (not shown) attachable to a distal end of the reservoir holder 3.

Figure 4 shows a sectional and perspective view of the mechanics holder wherein the cut runs along the longitudinal axis. As it can be seen in figure 4 the mechanics holder 10 has a sleeve-shaped body comprising a distal holder connecting portion 11 for a connection with the reservoir holder 3 and a proximal housing connecting portion 14 for a connection with a housing 51 of the manual drive unit 50.

In the middle the mechanics holder 10 includes a circumferential rim 13 separating the distal holder connecting portion 11 from the proximal housing connecting portion 14. In the holder connection portion 11 the mechanics holder 10 includes a nut 12 for a snapper of the reservoir holder 3. Hence the snapper and the nut 12 provide a non-releasable snap-fit connection when the holder connection portion 11 is inserted into an opening of the sleeve-shaped reservoir holder 3. The housing connecting 12 portion includes a protrusion or cam 15 in the middle part with respect to the longitudinal direction. If the reservoir unit 1 is used with a manual drive unit 50 the cam 15 can snap into a corresponding recess 41 on an inside of the housing 51 of the manual drive unit 50 thereby locking the mechanics holder 10 and thus the reservoir unit 1 to the manual drive unit 50. Alternatively, if the reservoir unit 1 is used with a reusable automatic drive unit 60 the cam 15 can be inserted into an L-shaped nut in a housing 62 of the drive unit 60 to form a releasable bayonet connection as described below. The proximal portion of the mechanics holder 10 is sleeve-shaped and extents in proximal direction. As shown in figure 1 the outer surface of the proximal portion comprises ribs or corrugates in order to provide a constant wall thickness, which is important for manufacturing by injection molding.

On its inside the mechanics holder 10 includes on a distal side an integrally formed (monolithic) biasing member connected to the body of the mechanics holder 10. The biasing member 18 comprises a compressible hollow chamber or recess and is made of the same plastic as the mechanics holder. The biasing member 18 is elastically deformable in proximal direction and is adapted to bias an inserted cartridge 4 in a distal direction. A radial wall in the middle of the mechanics holder includes two holes adapted to accommodate two holding arms of the biasing member 18.

In the axial middle part the mechanics holder 10 comprises an inner thread 17 for the plunger rod 30. Inside a proximal end portion of the mechanics holder a thread element 16 is arranged. The thread element 16 is adapted to provide a threaded engagement with a thread of a dose sleeve 53 of the manual drive unit 50. Furthermore, the inner radial wall including the plunger rod thread 17 acts as a dispensing stop element and limits a distal movement of the dose sleeve 53.

In an assembled state as shown in figure 5 the mechanics holder 10 is in-between the reservoir holder 3 and the housing 51 of the manual drive unit 50. Figure 5 is a length section of the manual injection device 5 shown in Fig. 2.

The manual drive unit 50 comprises the housing 51, the dose sleeve 53 coaxially arranged inside the housing 51, a drive sleeve 56 coaxially arranged inside the dose sleeve 53, a coupling ring 55, a stop nut 58 and a dispensing button 57 disposed at a proximal end of the housing 51.

A dose knob 54 is integral with dose sleeve 53. The dose sleeve 53 has the form of a hollow cylinder. On an outer surface of the dose sleeve 53 are helically arranged numbers which are visible through a dosage window in the housing 51 during dose setting and dispensing. In a distal portion the dose sleeve 53 comprises a dose insert 52 coaxially arranged within the dose sleeve 53. The dose insert 52 has a smaller diameter than the dose sleeve 53 and is fixedly connected or integral therewith. A cylindrical gap is present between an inner surface of the dose sleeve 53 and an outer surface of the dose insert 52. The proximal portion of the mechanics holder 10 of the reservoir unit 1 least partially protrudes into said gap. In the area of its distal end the dose insert 53 has an outer thread for threaded engagement with the thread element 16 of the mechanics holder 10. In a proximal portion, the dose sleeve 53 has axially extending notches in which the stop nut 58 is in splined engagement. The stop nut 58 is guided in axial direction and is rotationally coupled to the dose sleeve 53 by this interaction.

The drive sleeve 56 has a form of a hollow cylinder. In a distal portion the drive sleeve 56 is concentrically arranged around the plunger rod 30 of the reservoir unit, i.e. the plunger rod 30 is inserted into the drive sleeve 56 from a distal end thereof when the reservoir unit 1 is attached to the drive unit 50.

The plunger rod 30 is rotationally coupled to the drive sleeve 56 but freely slidable therein in the axial direction. This may be achieved by the provision of at least one axial nut 31 (figure 1) in the plunger rod 30 and an axial spline 59 (figure 5) on an inside of the drive sleeve 56. On a proximal portion the drive sleeve 56 includes an outer thread which is in threading engagement with the stop nut 58. At its proximal end the dispensing button 57 is inserted into the drive sleeve 56. Further, the drive sleeve 56 abuts on the dose sleeve 43 at its proximal end via a compressible coupling ring 55, which is located inside and on a proximal end of the dose sleeve 53.

To set a dose, the user grabs the dose set knob 54 and rotates it. This drives the dose sleeve 53 and the dose insert 52 into rotation. With this rotation, the dose sleeve 53 is screwed out of the housing 51, i.e. moved in the proximal direction by means of the threading engagement of the dose insert 52 with the mechanics holder 10. This movement of the dose sleeve 53 entrains a linear movement of the drive sleeve 56 in the proximal direction. However, the drive sleeve 56 is not rotated as it is rotationally fixed by means of a retaining member 40 arranged on a distal end thereof. The retaining member 40 itself is slidable relative to the mechanics holder 10 but rotationally fixed thereto by means of splines. As the dose sleeve 53 is rotated but the drive sleeve 56 is rotationally fix, the stop nut 58 moves up the outer thread of the proximal portion of the drive sleeve 56.

Once the dose is set, the user pushes the dispensing button 57 in the distal direction. As the dispensing button 57 is connected to the drive sleeve 56 the distal movement of the dispensing button 57 is transferred to the drive sleeve 56. This distal movement of the drive sleeve 56 has two effects: Firstly, the movement creates a pressure on the coupling ring 55 and compresses it which cause radial teeth arranged on the drive sleeve 56 to lock the coupling ring 55 and radial notches of the dose sleeve 43 to lock the coupling ring 55. Hence, the dose sleeve 43 and the drive sleeve 56 are rotationally coupled. Secondly, said movement also leads to a disengagement of the rotational coupling between the drive sleeve 56 and the retaining member 40 by a disengagement of protrusions located on the drive sleeve 56 from notches located on the retaining member 40.

When the dispensing button 57 is further pushed distally, the dose sleeve 53 begins to rotate through the threading engagement of the dose insert 52 with the proximal portion of the mechanics holder 10. As the dose sleeve 53 and the drive sleeve 56 are now rotationally coupled by the coupling ring 55, the drive sleeve 56 is driven in rotation as well. By the rotational coupling of the plunger rod 30 with the drive sleeve 56, the plunger rod 30 is also driven in rotation. As the plunger rod 30 is in threading engagement with the mechanics holder 10, the plunger rod 30 is screwed in the distal direction by this rotation. Hence, the plunger rod 30 may displace a piston (not shown) inside the cartridge 4 in the distal direction within the cartridge 4. Further, the retaining member 40 is also pushed in the distal direction.

Once the dose has been dispensed, i.e. the dose sleeve 53 has returned to its initial position as shown in figure 5, the user stops pushing on the dispensing button 57. The axial compression of the elastic coupling ring 55 is released causing a slight movement of the drive sleeve 56 relative to the dose sleeve 53 in the proximal direction, which leads the coupling ring 55 to release the rotational coupling of the drive sleeve 56 with the dose sleeve 53. Further, this movement also leads to the re-engagement of the rotational coupling between the drive sleeve 56 and the retaining member 40. The drug delivery device 1 is hence ready to dispense a further dose of the medicament.

As mentioned above figure 3 depicts a semi-disposable automatic injection device 6 with the disposable reservoir unit 1 and the reusable automatic drive unit 60. In contrast to the fully disposable injection device 5 described above (figure 2 and 5) the interface between the reservoir unit 1 and the automatic drive unit 60 is a releasably connection in the form of a bayonet connection. Hence, the user can attach the reservoir unit 1 to the drive unit 60 and detach the reservoir unit 1 in order to replace it. For this purpose, the cam 15 of the mechanics holder 10 can engage an L-shaped nut (not shown) on an inside of an opening in a housing 62 of the automatic drive unit 60. The cam 15 and the nut form thus a releasable bayonet connection. This is in contrast to the disposable injection pen (figure 2 and 5) where the reservoir unit 1 is non-releasably connected to the manual drive housing 51.

A shown in figure 3 the automatic drive unit 60 further includes a LCD unit with a display 61, a dispensing button 63, an electric motor, in particular a BLDC motor or stepper motor, for moving the plunger rod 30, a sleeve-shaped automatic drive member that can be releasably coupled to the plunger rod, a gear connecting the motor to the drive member, a printed circuit board with a controller for controlling the electric motor, an energy source in form of a rechargeable battery a data storage module and a communication module (all not shown).

During attachment of the reservoir unit 1 to the automatic drive unit 60 the plunger rod 30 is linearly inserted into the sleeve-shaped drive member and thus the plunger rod 30 is rotationally coupled to the drive member. With a twist movement of the reservoir unit 1 relative to the drive unit 60 the bayonet connection is locked. The automatic injection pen 6 is now ready for an injection. The user or a HCP can adjust injection settings on an external device (e. g. computer, smart phone, tablet, smart watch) and wirelessly transmit the injection data to the communication module in the drive unit 60. The user can start the injection by pressing the dispensing button.

In an alternative embodiment the automatic drive unit 60 may not comprise any display but only simple LEDs or no indication means at all. In this case the data are transmitted from the drive unit 60 to a user device such as a smart phone with a display for displaying the information.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | | |
|---|---|---|---|
| 1 | Reservoir unit | 40 | retaining member |
| 3 | Reservoir holder | 41 | recess |
| 4 | Cartridge | 50 | manual drive unit |
| 5 | Manual injection pen | 51 | housing |
| 6 | Automatic injection pen | 52 | dose insert |
| 10 | Mechanics holder | 53 | dose sleeve |
| 11 | holder coupling portion | 54 | dose knob |
| 12 | nut | 55 | coupling ring |
| 13 | rim | 56 | drive sleeve |
| 14 | housing coupling portion | 57 | dispensing button |
| 15 | cam | 58 | dose stop nut |
| 16 | thread element | 59 | spline |
| 17 | inner thread | 60 | automatic drive unit |
| 18 | biasing member | 61 | LCD |
| 30 | plunger rod | 62 | housing |
| 32 | thread | 63 | dispensing button |
| 31 | nut | | |
| 33 | flange | | |

## Claims

1. Drug delivery device (5, 6) for dispensing a drug from a reservoir (4), the delivery device comprising
- a drive unit (50, 60) including a housing (51, 62) defining a longitudinal axis and including a manual or automatic drive inside the housing (51, 62) with a movable drive member (56) for driving a plunger rod (30) for dispensing the drug,
- a pre-assembled reservoir unit (1) attachable to the drive unit (50, 60) along the longitudinal axis, wherein the reservoir unit (1) includes
the reservoir (4),
a reservoir holder (3) and
a mechanics holder (10) connected to the reservoir holder (3) and comprising a housing connecting portion (14) for coaxially attaching the mechanics holder (30) to the housing (51, 62) of the drive unit (50, 60)
**characterized in that**
the reservoir unit (1) includes the plunger rod (30), that is in threaded or splined engagement with the mechanics holder (10) and wherein
the drive member (56) includes a coupling structure (59) for coupling the drive member (56) to the plunger rod (30) such that a movement of the drive member (56) is transferred to the plunger rod (30) once the reservoir unit (1) is attached to the drive unit (50, 60).

2. Drug delivery device (5, 6) according to claim 1 wherein the mechanics holder (10) is made in one piece.

3. Drug delivery device (5, 6) according to claim 1 or 2 wherein the coupling structure (59) is a spline element for rotational coupling such that exclusively a rotational movement of the drive member (56) is transferred to the plunger rod (30), when the drive unit (50, 60) is attached to the reservoir unit (1).

4. Drug delivery device (5, 6) according to any of claim 1 to 3, wherein the mechanics holder (10) includes a snap-fit element and wherein the mechanics holder (10) is connectable to the reservoir holder (3) by a non-releasable snap-fit connection.

5. Drug delivery device (5, 6) according to any of claims 1 to 4 wherein the housing coupling portion (14) includes a first connecting element (15) on an outer surface of the mechanics holder (10) and wherein the housing (51) includes a second connecting element (41) arranged on an inside of an opening of the housing (51) and adapted to engage the first connecting (15) element and wherein the housing (51, 62) encloses at least a part of the mechanics holder (10), when the drive unit (50, 60) is attached to the reservoir unit (1).

6. Drug delivery device (5, 6) according to any of claims 1 to 5, wherein the mechanics holder (10) includes a thread (17) for the plunger rod (30).

7. Drug delivery device (5, 6) according to claim 1 to 6 wherein the mechanics holder (10) has a sleeve-shaped body including a distal end portion (11) for attachment to the reservoir holder (3) and a proximal portion for insertion into the housing (51, 62).

8. Drug delivery device (5) according to claim 7, wherein the mechanics holder (10) includes a proximally arranged dose thread (16) adapted to be in threaded connection with a dose member (53) of the drive unit (50).

9. Drug delivery device (5, 6) according to any of claims 1 to 8, wherein the mechanics holder (10) includes a biasing element (18) for biasing the reservoir (4) in a dispensing direction against the reservoir holder (3).

10. Drug delivery device (5, 6) according to claim 9, wherein the biasing element (18) is an integral part of the mechanics holder (10).

11. Drug delivery device (5, 6) according to any of claims 9 or 10, wherein the biasing element (18) includes a hollow chamber.

12. Drug delivery device (5, 6) according to any of claims 1 to 11, wherein the mechanics holder (30) is primarily made of a first plastic material and the housing (51, 62) is primarily made of a second plastic material different than the first plastic material.

13. Drug delivery device (6) according to any of claims 1 to 12, wherein the drive unit (60) is an automatic drive unit and includes a motor or a pre-tensioned or a re-tensionable member for automatically moving the drive member.

14. Drug delivery device (5) according to any of claims 1 to 12, wherein the drive unit is a manual drive unit (50) and includes a dose member (53) for manually setting a dose and for manually rotating to dispense the dose and wherein the dose member (53) is selectively connectable to the drive member (56) for dose dispensing.

15. A pre-assembled reservoir unit (1) for attachment to a drive unit (50, 60) of a drug delivery device (5, 6) for dispensing a drug from a reservoir (4), the reservoir unit (1) comprises
the reservoir (4),
a reservoir holder (3) and
a mechanics holder (10) defining a longitudinal axis and connected to the reservoir holder (3), wherein the mechanics holder (10) comprises a housing connecting portion (14) for coaxially attaching the mechanics holder (10) to a housing (51, 62) of the drive unit along the longitudinal direction
**characterized in that**
the reservoir unit (1) includes a plunger rod (30) that is in threaded or splined engagement with the mechanics holder (10).
